# EUROPEAN PATENT APPLICATION

(11) **EP 4 826 386 A1**
(43) Date of publication of application: **21.10.2026**
(21) Application number: 24907093.9
(22) Date of filing: 27.11.2024
(51) Int. Cl.: G01N 33/68

(54) **METHOD FOR USING PRESCRIBED QUANTITATIVE VALUE OF COLLAGEN CONTAINED IN TEETH OF MAMMAL**

(30) Priority: 19.12.2023 JP 2023213847
(71) Applicant: Devine Inc., Sapporo-shi, Hokkaido 060-0807 (JP)
(72) Inventor: OKUBO Naoto, Sapporo-shi, Hokkaido 060-0808 (JP); HOSONO Hidetaka, Sapporo-shi, Hokkaido 060-0807 (JP)
(74) Representative: Prüfer & Partner mbB Patentanwälte · Rechtsanwälte
(86) International application number: PCT/JP2024/041899
(87) International publication number: WO 2025/134705

(57) **Abstract**

[Problem] To provide a method for using a prescribed quantitative value and/or weight ratio value of collagen contained in a tooth of a mammal as an index for evaluating a potential risk of developing a disease in the mammal and/or whether the mammal is potentially healthy.

[Solution] At least one of PYD, PEN, T-GLYC, PpP, and PpG is compared with a reference value thereof with respect to each of: collagen contained in a tooth of a mammal to be evaluated for a potential risk of developing a disease and/or whether the mammal is potentially healthy; and collagen contained in a tooth of one or more mammals selected from a mammal that has not developed a disease, a mammal that has developed a disease, a healthy mammal, and a non-healthy mammal.

## Description

### [Technical Field]

The present invention relates to at least any one method, as an index for evaluating a potential risk of developing a disease in a mammal and/or whether the mammal is potentially healthy, of a method of using a quantitative value of pyridinoline content (ng/mg) of collagen contained in teeth of a mammal, a method of using a quantitative value of pentosidine content (ng/mg) of collagen contained in teeth of a mammal, a method of using a quantitative value of total glycation product content (ng/mg: converted to pentosidine content), a method of using a value of a weight ratio (pyridinoline content (ng/mg)/ pentosidine content (ng/mg)) calculated from a quantitative value of pyridinoline content (ng/mg) of collagen contained in teeth of a mammal and a quantitative value of pentosidine content (ng/mg) thereof, as well as a method of using a value of a weight ratio (pyridinoline content (ng/mg)/ total glycation product content (ng/mg: converted to pentosidine content)) calculated from a quantitative value of pyridinoline content (ng/mg) of collagen contained in teeth of a mammal and a quantitative value of total glycation product content (ng/mg: converted to pentosidine content) thereof.

### [Background Art]

Conventionally, various collagens (in reality, they are a "collagen-rich organic composition matter" correctly, but they are often referred to as simply "collagen" in general) have been developed. Almost all of them are an atelocollagen manufactured by being extracted, through using an enzyme treatment method, from a soft tissue within a living body, and are a collagen wherein telopeptides present respectively at an N-terminus and a C-terminus in an amino acid sequence of collagen has been severed and simultaneously wherein hardly-soluble collagen fibers have been eliminated by means of a purification process. Additionally, collagens manufactured by being extracted from a hard tissue such as bones and teeth, such as DFDBA (Demineralized Freeze-Dried Bone Allograft) and DDM (Demineralized Dentin Matrix) have been also developed, and collagens are manufactured by means of demineralization treatment and are mainly used for biomaterials.

Atelocollagen is evaluated in quality of collagen with such a matter as a telopeptide said to have antigenicity has been severed by means of enzyme treatment being a reason, whereas non-denatured collagen (collagen having a triple helix structure because it has telopeptide retained) derived from a soft tissue is evaluated in quality of collagen on a basis of how high in purity the collagen is manufactured by means of a condition setting for purification treatment, such as pH. Incidentally, in a case of manufacturing collagen from a hard tissue, it is necessary to extract collagen through eliminating minerals by means of demineralization treatment, that is, acid treatment, which makes collagen limited in yield and besides, laborious to extract. Therefore, it has resulted that almost all collagen products being developed are atelocollagen and non-denatured collagen, derived from a soft tissue, or products utilizing such.

Almost all collagens contained in living tissues are a type I collagen. In a type I collagen, a physiological crosslinking and a nonphysiological crosslinking are present, and as a physiological crosslinking molecule, a pyridinoline crosslinking molecule is known and as a nonphysiological crosslinking molecule, a crosslinking molecule based on advanced glycation end-products (AGEs) represented by pentosidine is known, for each (Non-patent Literature Document 1). Since a pyridinoline crosslinking is a physiological/ enzymatic crosslinking based on action of lysyl oxidase and a regular crosslinking molecule in a specific site in an amino acid sequence of collagen, it contributes to enhancement of elasticity and strength of collagen fibers. Meanwhile, since a pentosidine crosslinking is a nonphysiological/ nonenzymatic crosslinking (aging crosslinking (AGEs crosslinking)) related to by blood glucose and is a crosslinking molecule formed between arginine and lysine residues scattered in an amino acid sequence of collagen due to blood glucose being warmed by body temperature, it is a crosslinking molecule randomly formed at arginine and lysine residues near to each other in an amino acid sequence of collagen and is a factor of disturbing a physiological steric structure of collagen, and contributes to deterioration of elasticity and strength of collagen fibers. Since atelocollagen has, as described above, its telopeptides severed through enzyme treatment, it is a collagen not only containing almost no pyridinoline crosslinking molecules but also containing almost no pentosidine crosslinking molecules because hardly-soluble collagen fibers caused by pentosidine crosslinking are eliminated by means of a purification process.

Conventionally, it has been known that by pyridinoline crosslinking molecules in collagen being increasing, that collagen increases in strength and elasticity (Non-patent Literature Document 2), and besides, it has been known that pentosidine crosslinking is a pathological crosslinking (aging crosslinking (AGEs crosslinking)) (Non-patent Literature Document 3). Additionally, it has been reported that pentosidine quantity per unit collagen in human articular cartilage increases linearly with age, and also that increase of a ratio of pentosidine quantity and pyridinoline quantity per that unit collagen (pentosidine content/ pyridinoline content) is accelerated with age (Non-patent Literature Documents 4 and 5), and it has been reported that a ratio of pentosidine content and pyridinoline content per that unit collagen (pentosidine content/ pyridinoline content) increases significantly in noncalcified lesions of dystrophic calcification of aortae (Non-patent Literature Document 6).

### [Prior Art Documents]

### [Non-patent Literature Document]

[Non-patent Literature Document 1]
   M. Saito, CLINICIAN Vol.554, 1141-1146, 2006
[Non-patent Literature Document 2]
   M. Saito, THE BONE Vol.21, No.1, 53-58, 2007
[Non-patent Literature Document 3]
   Sell, D.R.et al., V.M., J. Biol. Chem. Vol.284, 21597-21602,1989
[Non-patent Literature Document 4]
   A. Uchiyama et al., J. Biochem. Vol.110, 714-718, 1991
[Non-patent Literature Document 5]
   M. TAKAHASHI et al., Arthritis Rheum. Vol.37, No.5, 724-728, May 1994.
[Non-patent Literature Document 6]
   H. HOSHINO et al., Atherosclerosis Vol.112, 39-46, 1995

### [Summary of Invention]

### [Technical Problem]

Since the above-mentioned evaluation on quality of collagen (collagen-rich organic composition matter) is, by and large, made in accordance with a purity degree and origin of collagen in a collagen-rich organic composition matter and is not made on a basis of some molecule-level index of collagen, that is, some amino acid sequence-level index of collagen, it is hard to say that adequate evaluation has been made on quality of collagen in itself, and furthermore, evaluation on quality of collagen has not been made on a basis of pyridinoline content, which contributes to enhancement of quality of collagen, such as enhancement of elasticity and strength of collagen fibers, pentosidine content and total glycation product content, which is a factor of disturbing a physiological steric structure of collagen and causes deterioration of elasticity and strength of collagen fibers, and ultimately contributes to deterioration of quality of collagen, a weight ratio of pyridinoline content and pentosidine content. as well as a weight ratio of pyridinoline content and total glycation product content.

Additionally, certainly in Non-patent Literature Documents 1~6 above, pyridinoline crosslinking molecules and pentosidine crosslinking molecules in collagen are variously mentioned, and besides, ratios of pentosidine content and pyridinoline content per unit collagen (pentosidine content/ pyridinoline content) in diseases are considered, but none of the literature documents is not in particular about tooth-derived collagen wherein the collagen cannot be easily extracted. In addition, although in tissues physiologically requiring strength like hard tissues such as bones and cartilages, and soft tissues such as tendons and ligaments, a quantity of pyridinoline crosslinking molecules contained in collagen contained and accordingly, a quantity of pentosidine crosslinking molecules or total glycation product crosslinking molecules tends to be a large quantity (M. TAKAHASHI et al., Anal Biochem Vol. 232, 158-62, 1995), those tissues are tissues to be metabolized, and therefore, pyridinoline crosslinking molecules, and pentosidine crosslinking molecules, total glycation product crosslinking molecules, contained therein are also metabolized so that it does not follow that a total accumulation quantity of pyridinoline crosslinking molecules, and pentosidine crosslinking molecules, total glycation product crosslinking molecules, which have been so far accumulated in a host being a mammal is reflected. In contrast, since teeth being hard tissues are sole tissues not being incorporated in metabolism cycles among hard tissues, pyridinoline crosslinking molecules, and pentosidine crosslinking molecules, total glycation product crosslinking molecules, contained in that collagen contained are not metabolized, and therefore, it follows that a total accumulation quantity of pyridinoline crosslinking molecules, and pentosidine crosslinking molecules, total glycation product crosslinking molecules, which have been so far accumulated in a host being a mammal is reflected. Accordingly, although it is clear that for accurately evaluating a potential risk of developing a disease in a mammal and/or whether the mammal is potentially healthy, tooth-derived collagen (a collagen-rich organic composition matter) wherein a total accumulation quantity of pyridinoline crosslinking molecules, and pentosidine crosslinking molecules, total glycation product crosslinking molecules, which have been so far accumulated in a host being a mammal is reflected should be made to be an index, the Non-patent Literature Documents 1~6 above not only do not describe, but also do not even suggest such.

The present invention is intended to provide any one of a method which is a method of using, as an index for evaluating a potential risk of developing a disease in a mammal and/or whether the mammal is potentially healthy, a quantitative value of pyridinoline content of collagen contained in teeth of a mammal, and which includes a process step of obtaining a PYD being a quantitative value by quantifying pyridinoline content (ng/mg) of collagen contained in teeth of a mammal to be evaluated for a potential risk of developing a disease and/or whether the mammal is potentially healthy, a process step of obtaining an R-PYD being a reference quantitative value by quantifying pyridinoline content (ng/mg) of collagen contained in teeth of one or more mammals selected from a mammal that has not developed a disease, a mammal that has developed a disease, a healthy mammal, and a non-healthy mammal, and a process step of comparing said PYD with said R-PYD, a method which is a method of using, as an index for evaluating a potential risk of developing a disease in a mammal and/or whether the mammal is potentially healthy, a quantitative value of pentosidine content of collagen contained in teeth of a mammal, and which includes a process step of obtaining a PEN being a quantitative value by quantifying pentosidine content (ng/mg) of collagen contained in teeth of a mammal to be evaluated for a potential risk of developing a disease and/or whether the mammal is potentially healthy, a process step of obtaining an R-PEN being a reference quantitative value by quantifying pentosidine content (ng/mg) of collagen contained in teeth of one or more mammals selected from a mammal that has not developed a disease, a mammal that has developed a disease, a healthy mammal, and a non-healthy mammal, and a process step of comparing said PEN with said R-PEN, a method which is a method of using, as an index for evaluating a potential risk of developing a disease in a mammal and/or whether the mammal is potentially healthy, a quantitative value of total glycation product content of collagen contained in teeth of a mammal, and which includes a process step of obtaining a T-GLYC being a quantitative value by quantifying total glycation product content (ng/mg: converted to pentosidine content) of collagen contained in teeth of a mammal to be evaluated for a potential risk of developing a disease and/or whether the mammal is potentially healthy, a process step of obtaining an R-T-GLYC being a reference quantitative value by quantifying total glycation product content (ng/mg: converted to pentosidine content) of collagen contained in teeth of one or more mammals selected from a mammal that has not developed a disease, a mammal that has developed a disease, a healthy mammal, and a non-healthy mammal, and a process step of comparing said T-GLYC with said R-T-GLYC, a method which is a method of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of pentosidine content thereof, and which includes a process step of obtaining a PYD being a quantitative value by quantifying pyridinoline content (ng/mg) of collagen contained in teeth of a mammal to be evaluated for a potential risk of developing a disease and/or whether the mammal is potentially healthy, a process step of obtaining a PEN being a quantitative value by quantifying pentosidine content (ng/mg) of collagen contained in teeth of said mammal to be evaluated, a process step of calculating, from said PYD obtained and said PEN obtained, a PpP (pyridinoline content (ng/mg)/ pentosidine content (ng/mg)) being a value of a weight ratio, a process step of obtaining an R-PYD being a reference quantitative value by quantifying pyridinoline content (ng/mg) of collagen contained in teeth of one or more mammals selected from a mammal that has not developed a disease, a mammal that has developed a disease, a healthy mammal, and a non-healthy mammal, a process step of obtaining an R-PEN being a reference quantitative value by quantifying pentosidine content (ng/mg) of collagen contained in teeth of said one or more mammals, a process step of calculating, from said R-PYD obtained and said R-PEN obtained, an R-PpP (pyridinoline content (ng/mg)/ pentosidine content (ng/mg)) being a reference value of a weight ratio, and a process step of comparing said PpP with said R-PpP, as well as a method which is a method of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of total glycation product content thereof, and which includes a process step of obtaining a PYD being a quantitative value by quantifying pyridinoline content (ng/mg) of collagen contained in teeth of a mammal to be evaluated for a potential risk of developing a disease and/or whether the mammal is potentially healthy, a process step of obtaining a T-GLYC being a quantitative value by quantifying total glycation product content (ng/mg: converted to pentosidine content) of collagen contained in teeth of said mammal to be evaluated, a process step of calculating, from said PYD obtained and said T-GLYC obtained, a PpG (pyridinoline content (ng/mg)/ total glycation product content (ng/mg: converted to pentosidine content)) being a value of a weight ratio, a process step of obtaining an R-PYD being a reference quantitative value by quantifying pyridinoline content (ng/mg) of collagen contained in teeth of one or more mammals selected from a mammal that has not developed a disease, a mammal that has developed a disease, a healthy mammal, and a non-healthy mammal, a process step of obtaining an R-T-GLYC being a reference quantitative value by quantifying total glycation product content (ng/mg: converted to pentosidine content) of collagen contained in teeth of said one or more mammals, a process step of calculating, from said R-PYD obtained and said R-T-GLYC obtained, an R-PpG (pyridinoline content (ng/mg)/ total glycation product content (ng/mg: converted to pentosidine content)) being a reference value of a weight ratio, and a process step of comparing said PpG with said R-PpG.

### [Solution to Problem]

Inventors of the present invention have found out, as a result of extensive research to solve the above problems, that by comparing at least any one value of the above-mentioned PYD, PEN, T-GLYC, PpP, and PpG for each of collagen contained in teeth of a mammal to be evaluated for a potential risk of developing a disease and/or whether the mammal is potentially healthy and collagen contained in teeth of one or more mammals selected from a mammal that has not developed a disease, a mammal that has developed a disease, a healthy mammal, and a non-healthy mammal, with a reference value of each of them, a potential risk of developing a disease in a mammal and/or whether the mammal is potentially healthy can be evaluated, and have accomplished each of the inventions below.
(1) A method which is a method of using, as an index for evaluating a potential risk of developing a disease in a mammal and/or whether the mammal is potentially healthy, a quantitative value of pyridinoline content of collagen contained in teeth of a mammal, and which includes a process step of obtaining a PYD being a quantitative value by quantifying pyridinoline content (ng/mg) of collagen contained in teeth of a mammal to be evaluated for a potential risk of developing a disease and/or whether the mammal is potentially healthy, a process step of obtaining an R-PYD being a reference quantitative value by quantifying pyridinoline content (ng/mg) of collagen contained in teeth of one or more mammals selected from a mammal that has not developed a disease, a mammal that has developed a disease, a healthy mammal, and a non-healthy mammal, and a process step of comparing said PYD with said R-PYD.
(2) A method which is a method of using, as an index for evaluating a potential risk of developing a disease in a mammal and/or whether the mammal is potentially healthy, a quantitative value of pentosidine content of collagen contained in teeth of a mammal, and which includes a process step of obtaining a PEN being a quantitative value by quantifying pentosidine content (ng/mg) of collagen contained in teeth of a mammal to be evaluated for a potential risk of developing a disease and/or whether the mammal is potentially healthy, a process step of obtaining an R-PEN being a reference quantitative value by quantifying pentosidine content (ng/mg) of collagen contained in teeth of one or more mammals selected from a mammal that has not developed a disease, a mammal that has developed a disease, a healthy mammal, and a non-healthy mammal, and a process step of comparing said PEN with said R-PEN.
(3) A method which is a method of using, as an index for evaluating a potential risk of developing a disease in a mammal and/or whether the mammal is potentially healthy, a quantitative value of total glycation product content of collagen contained in teeth of a mammal, and which includes a process step of obtaining a T-GLYC being a quantitative value by quantifying total glycation product content (ng/mg: converted to pentosidine content) of collagen contained in teeth of a mammal to be evaluated for a potential risk of developing a disease and/or whether the mammal is potentially healthy, a process step of obtaining an R-T-GLYC being a reference quantitative value by quantifying total glycation product content (ng/mg: converted to pentosidine content) of collagen contained in teeth of one or more mammals selected from a mammal that has not developed a disease, a mammal that has developed a disease, a healthy mammal, and a non-healthy mammal, and a process step of comparing said T-GLYC with said R-T-GLYC.
(4) A method which is a method of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of pentosidine content thereof, and which includes a process step of obtaining a PYD being a quantitative value by quantifying pyridinoline content (ng/mg) of collagen contained in teeth of a mammal to be evaluated for a potential risk of developing a disease and/or whether the mammal is potentially healthy, a process step of obtaining a PEN being a quantitative value by quantifying pentosidine content (ng/mg) of collagen contained in teeth of said mammal to be evaluated, a process step of calculating, from said PYD obtained and said PEN obtained, a PpP (pyridinoline content (ng/mg)/ pentosidine content (ng/mg)) being a value of a weight ratio, a process step of obtaining an R-PYD being a reference quantitative value by quantifying pyridinoline content (ng/mg) of collagen contained in teeth of one or more mammals selected from a mammal that has not developed a disease, a mammal that has developed a disease, a healthy mammal, and a non-healthy mammal, a process step of obtaining an R-PEN being a reference quantitative value by quantifying pentosidine content (ng/mg) of collagen contained in teeth of said one or more mammals, a process step of calculating, from said R-PYD obtained and said R-PEN obtained, an R-PpP (pyridinoline content (ng/mg)/ pentosidine content (ng/mg)) being a reference value of a weight ratio, and a process step of comparing said PpP with said R-PpP.
(5) A method which is a method of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of total glycation product content thereof, and which includes a process step of obtaining a PYD being a quantitative value by quantifying pyridinoline content (ng/mg) of collagen contained in teeth of a mammal to be evaluated for a potential risk of developing a disease and/or whether the mammal is potentially healthy, a process step of obtaining a T-GLYC being a quantitative value by quantifying total glycation product content (ng/mg: converted to pentosidine content) of collagen contained in teeth of said mammal to be evaluated, a process step of calculating, from said PYD obtained and said T-GLYC obtained, a PpG (pyridinoline content (ng/mg)/ total glycation product content (ng/mg: converted to pentosidine content)) being a value of a weight ratio, a process step of obtaining an R-PYD being a reference quantitative value by quantifying pyridinoline content (ng/mg) of collagen contained in teeth of one or more mammals selected from a mammal that has not developed a disease, a mammal that has developed a disease, a healthy mammal, and a non-healthy mammal, a process step of obtaining an R-T-GLYC being a reference quantitative value by quantifying total glycation product content (ng/mg: converted to pentosidine content) of collagen contained in teeth of said one or more mammals, a process step of calculating, from said R-PYD obtained and said R-T-GLYC obtained, an R-PpG (pyridinoline content (ng/mg)/ total glycation product content (ng/mg: converted to pentosidine content)) being a reference value of a weight ratio, and a process step of comparing said PpG with said R-PpG.
(6) A method according to (1), wherein a process step of carrying out quantification is a process step of carrying out quantification by utilizing a high-performance liquid chromatograph with a fluorescence detector (HPLC-Flu) using heptafluorobutyric acid and formic acid as an ion-pairing reagent.
(7) A method according to any one of (1) to (6), wherein said mammals are one or more mammals selected from a group consisting of humans, bovines, horses, swine, wild boars, sheep, deer, dogs, and cats.

### [Effect of Invention]

By the present invention, a potential risk of developing a disease in a mammal and/or whether the mammal is potentially healthy is allowed to be evaluated by comparing at least any one value of the above-mentioned PYD, PEN, T-GLYC, PpP, and PpG with a reference value for each of them, which are, respectively, for collagen contained in teeth of a mammal to be evaluated for a potential risk of developing a disease and/or whether the mammal is potentially healthy and collagen contained in teeth of one or more mammals selected from a mammal that has not developed a disease, a mammal that has developed a disease, a healthy mammal, and a non-healthy mammal.

### [Brief Description of Drawings]

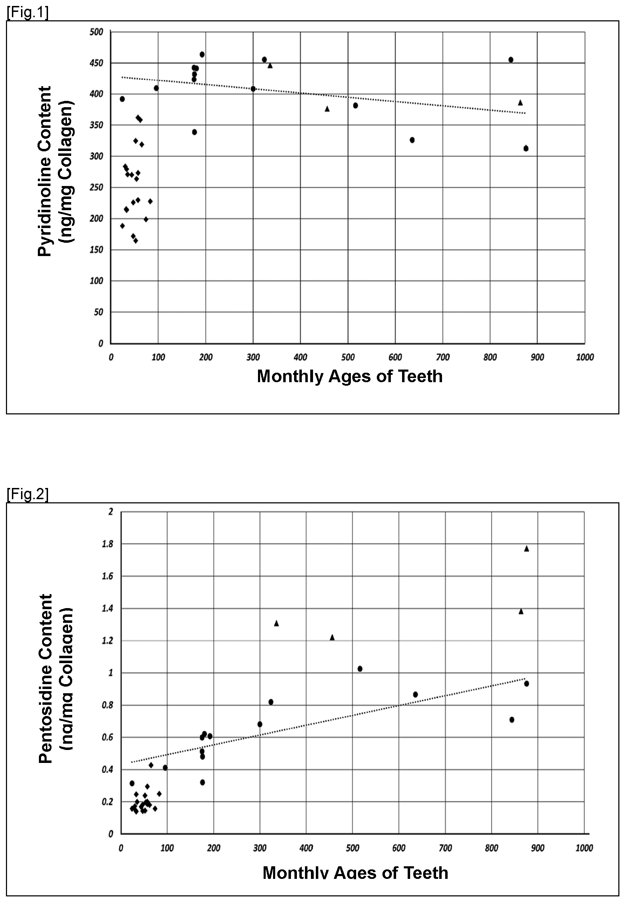

[Fig.1] A distribution diagram according to monthly ages of teeth, of a PYD being a quantitative value of pyridinoline content (ng/mg) quantified by means of a high-performance liquid chromatograph with a fluorescence detector (HPLC-Flu) using heptafluorobutyric acid and formic acid as an ion-pairing reagent, of hydrolysis products of collagen contained in teeth (37 cases in total) of human non-diabetic patients 14 cases (•), human diabetic patients 4 cases (▲), and bovines ((Specified Risk Materials; SRM) of bovines) 19 cases (◆). In the diagram, a broken line represents an approximation curve (linear approximation) based on PYDs of human non-diabetic patients 14 cases, which correspond to an R-PYD.
[Fig.2] A distribution diagram according to monthly ages of teeth, of a PEN being a quantitative value of pentosidine content (ng/mg) quantified by means of a high-performance liquid chromatograph with a fluorescence detector (HPLC-Flu) using heptafluorobutyric acid and formic acid as an ion-pairing reagent, of hydrolysis products of collagen contained in teeth (37 cases in total) of human non-diabetic patients 14 cases (●), human diabetic patients 4 cases (▲), and bovines ((Specified Risk Materials; SRM) of bovines) 19 cases (◆). In the diagram, a broken line represents an approximation curve (linear approximation) based on PENs of human non-diabetic patients 14 cases, which correspond to an R-PEN.

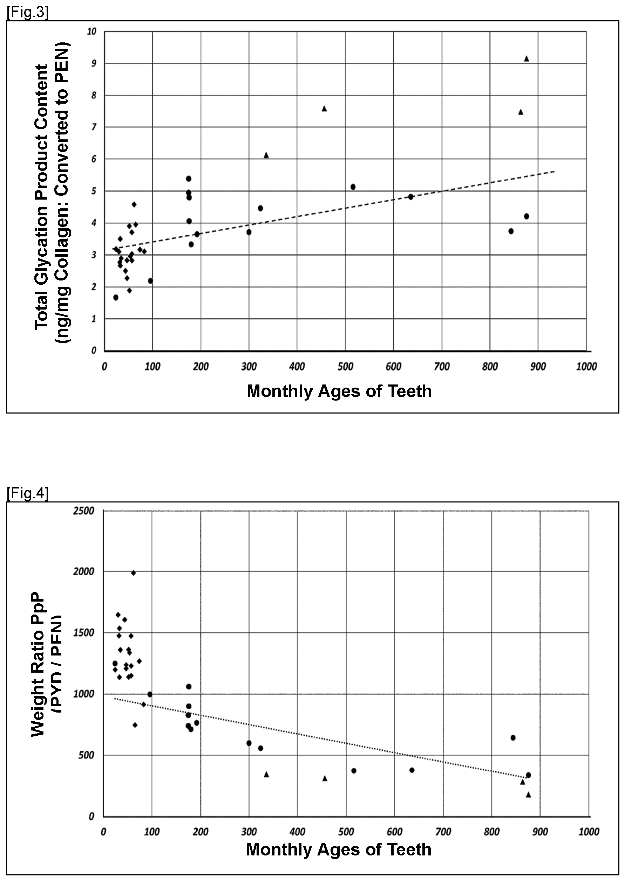

[Fig.3] A distribution diagram according to monthly ages of teeth, of a T-GLYC being a quantitative value of total glycation product content (ng/mg: converted to pentosidine content) quantified by means of a high-performance liquid chromatograph with a fluorescence detector (HPLC-Flu) using heptafluorobutyric acid and formic acid as an ion-pairing reagent, of hydrolysis products of collagen contained in teeth (37 cases in total) of human non-diabetic patients 14 cases (●), human diabetic patients 4 cases (▲), and bovines ((Specified Risk Materials; SRM) of bovines) 19 cases (◆). In the diagram, a broken line represents an approximation curve (linear approximation) based on T-GLYCs of human non-diabetic patients 14 cases, which correspond to an R-T-GLYC.
[Fig.4] A distribution diagram according to monthly ages of teeth, of a PpP (pyridinoline content (ng/mg)/ pentosidine content (ng/mg)) being a value of a weight ratio calculated from a PYD being a quantitative value of pyridinoline content (ng/mg) and a PEN being a quantitative value of pentosidine content (ng/mg), which were determined through quantification by means of a high-performance liquid chromatograph with a fluorescence detector (HPLC-Flu) using heptafluorobutyric acid and formic acid as an ion-pairing reagent, of hydrolysis products of collagen contained in teeth (37 cases in total) of human non-diabetic patients 14 cases (●), human diabetic patients 4 cases (▲), and bovines ((Specified Risk Materials; SRM) of bovines) 19 cases (◆). In the diagram, a broken line represents an approximation curve (linear approximation) based on PpPs of human non-diabetic patients 14 cases, which correspond to an R-PpP.

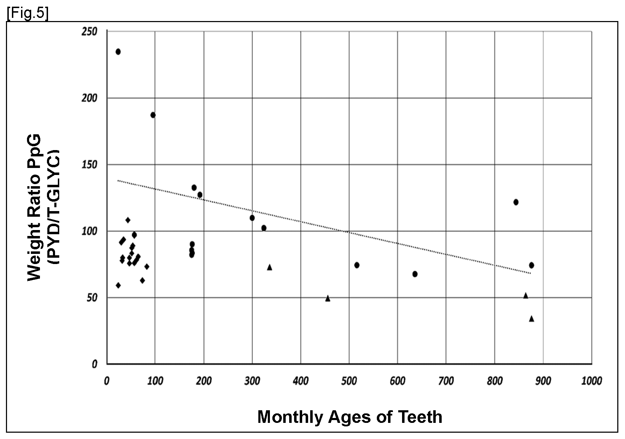

[Fig.5] A distribution diagram according to monthly ages of teeth, of a PpG (pyridinoline content (ng/mg)/ total glycation product content (ng/mg: converted to pentosidine content)) being a value of a weight ratio calculated from a PYD being a quantitative value of pyridinoline content (ng/mg) and a T-GLYC being a quantitative value of total glycation product content (ng/mg: converted to pentosidine content), which were determined through quantification by means of a high-performance liquid chromatograph with a fluorescence detector (HPLC-Flu) using heptafluorobutyric acid and formic acid as an ion-pairing reagent, of hydrolysis products of collagen contained in teeth (37 cases in total) of human non-diabetic patients 14 cases (●), human diabetic patients 4 cases (▲), and bovines ((Specified Risk Materials; SRM) of bovines) 19 cases (◆). In the diagram, a broken line represents an approximation curve (linear approximation) based on PpGs of human non-diabetic patients 14 cases, which correspond to an R-PpG.

### [Description of Embodiments]

A method according to the present invention, of using, as an index for evaluating a potential risk of developing a disease in a mammal and/or whether the mammal is potentially healthy, a quantitative value of pyridinoline content (ng/mg) of collagen contained in teeth of a mammal, a method according to the present invention, of using, as an index for evaluating a potential risk of developing a disease in a mammal and/or whether the mammal is potentially healthy, a quantitative value of pentosidine content (ng/mg) of collagen contained in teeth of a mammal, a method according to the present invention, of using, as an index for evaluating a potential risk of developing a disease in a mammal and/or whether the mammal is potentially healthy, a quantitative value of total glycation product content (ng/mg: converted to pentosidine content) of collagen contained in teeth of a mammal, a method according to the present invention, of using, as an index for evaluating a potential risk of developing a disease in a mammal and/or whether the mammal is potentially healthy, a value of a weight ratio (pyridinoline content (ng/mg)/ pentosidine content (ng/mg)) calculated from a quantitative value of pyridinoline content (ng/mg) of collagen contained in teeth of a mammal and a quantitative value of pentosidine content (ng/mg) thereof, as well as a method according to the present invention, of using, as an index for evaluating a potential risk of developing a disease in a mammal and/or whether the mammal is potentially healthy, a value of a weight ratio (pyridinoline content (ng/mg)/ total glycation product content (ng/mg: converted to pentosidine content)) calculated from a quantitative value of pyridinoline content (ng/mg) of collagen contained in teeth of a mammal and a quantitative value of total glycation product content (ng/mg: converted to pentosidine content) thereof are described in detail, below. A numerical range represented by using "~" or ''from" in the present description means a range including, as its lower limit value and its upper limit value, numerical values stated before and after the "~" or "from".

In the present invention, "mammal" refers to basically an animal which reproduces sexually, is viviparous for many species existing presently, and feeds its children with breast milk, and when an animal is such, it is not limited in particular, and as such an animal, for example, humans; apes such as orangutans, gorillas, chimpanzees, bonobos, and monkeys belonging to gibbon family; large mammals such as, monkeys other than those belonging to gibbon family, and besides, bovines, horses, swine, wild boars, sheep, goats, deer, antelopes, bears, sea lions, seals, walruses, northern sea lions, fur seals, and whales; small mammals such as dogs, cats, rabbits, rats, squirrels, weasels, raccoons, and mongooses, can be mentioned, and humans, bovines, horses, swine, wild boars, sheep, deer, dogs, and cats can be mentioned as preferable mammals, and humans, bovines, horses, swine, dogs, and cats can be mentioned as more preferable mammals, and humans, bovines, dogs, and cats can be mentioned as most preferable mammals.

In the present invention, "potential risk of developing" does not indicate a state of developing a disease, but refers to a state where a risk is present, such as host-side defense reaction works maximally against a disease so that disease development is barely kept from coming true, and means a state where even in subjective findings, objective findings, and conventional general examination findings, it can be hardly checked that a disease is developed.

Additionally, in the present invention, "potentially healthy" refers to such a state as falls short of a non-healthy state, and means a state where even in subjective findings, objective findings, and conventional general examination findings, it can be hardly checked to be not healthy, whereas in the present invention, "potentially non-healthy" refers to such a state as falls short of a healthy state, and means a state where even in subjective findings, objective findings, and conventional general examination findings, it can be hardly checked to be healthy.

Collagen is one protein mainly constituting various organs, blood vessels, nerves, skins, ligaments, tendons, bones, cartilages, dental dentins, and so on of a vertebrate and made to be a framework of various tissues within an living body thereof, and is a main constituent of an extracellular matrix of a multicellular animal. In the present invention, "collagen" mainly refers to type I collagen. Additionally, since "collagen" of the present invention is collagen of teeth while teeth are tissues on which strong load called bite force is imposed, the collagen of teeth is, unlike that of soft tissues, required to have remarkably high strength by which it has to endure bite force.

Hard-tissue collagen can, with BMP-2, BMP-4,BMP-7, or osteocalcin being detected, be determined to derive from hard tissues, and can, with dentin sialoprotein (DSP), dentin glycoprotein (DGP), or dentin phosphoprotein (DPP) being detected, be determined to derive from teeth, for example.

In the present invention, contained pyridinoline, contained pentosidine, and contained total glycation products, of collagen are, respectively, a pyridinoline constituting pyridinoline crosslinking molecules being physiological crosslinking molecules in collagen, a pentosidine constituting pentosidine crosslinking molecules being nonphysiological crosslinking molecules in collagen, and total glycation products constituting total glycation product crosslinking molecules being nonphysiological crosslinking molecules in collagen. In the present invention, through quantifying respectively pyridinoline content (ng/mg) of collagen, pentosidine content (ng/mg) of collagen, and total glycation product content (ng/mg: converted to pentosidine content) of collagen, a PYD, a PEN, and a T-GLYC being quantitative values are obtained, and additionally, through dividing the present PYD by the present PEN, a PpP (pyridinoline content (ng/mg)/ pentosidine content (ng/mg)) being a value of a weight ratio of the present PYD to the present PEN has been, and through dividing the present PYD by the present T-GLYC, a PpG (pyridinoline content (ng/mg)/ total glycation product content (ng/mg: converted to pentosidine content)) being a value of a weight ratio of the present PYD to the present T-GLYC has been, each calculated.

It is not too much to say that a pyridinoline crosslinking being of a physiological/ enzymatic crosslinking molecule has been programmed with its purpose being mainly to enhance fundamental strength of a hard tissue, and as stated above, it is said to be a crosslinking molecule formed, by means of enzymatic action of lysyl oxidase, between two lysine residues in telopeptides present respectively at an N-terminus and a C-terminus in an amino acid sequence of collagen, and one lysine residue in a amino acid sequence excluding both termini of collagen. Since a pyridinoline crosslinking molecule formed at two lysine residues in telopeptides present respectively at an N-terminus and a C-terminus in an amino acid sequence of collagen is a crosslinking molecule between collagen fibers, it can be said to greatly contribute to maintaining a steric structure property of a tropocollagen (whole collagen sequence) which is in a triple helix structure.

Therefore, when pyridinoline crosslinking molecules increase in collagen contained in a living tissue, they make quality of collagen contained in that living tissue be high in suppleness and consistency, and enhance strength and calcification degree of the collagen, and can be an element of enhancing activity of cells constituting that living tissue, that is, by activity of tissue stem cells involved in tissue repair being elevated, be an element of making homeostasis of an individual be easy to maintain. Therefore, since collagen derived from teeth or bones is, as compared with collagen of soft-tissues represented by skins, great in pyridinoline content, the collagen derived from teeth or bones is supple, high in strength, and high in elasticity.

Meanwhile, crosslinking by pentosidine crosslinking molecules being nonphysiological crosslinking molecules and advanced glycation end-products (AGEs) represented by total glycation product crosslinking molecules including pentosidine crosslinking molecules is a pathological crosslinking (aging crosslinking (AGEs crosslinking)) caused by blood glucose, and is, as described above, a crosslinking molecule formed, due to blood glucose being warmed by body temperature, and due to various combinations at arginine and lysine residues scattered in an amino acid sequence of collagen. Since in a case where arginine and arginine residues, lysine and lysine residues, and, arginine and lysine residues in an amino acid sequence of collagen are near to each other, pentosidine crosslinking molecules and total glycation product crosslinking molecules including pentosidine crosslinking molecules are formed nonspecifically (randomly), they can put collagen fibers in a state of being entangled, and are a factor of making arrangement regularity of collagen fibers be lost and disturbing a steric structure of tropocollagen, and therefore, a matter that pentosidine crosslinking molecules and total glycation product crosslinking molecules including pentosidine crosslinking molecules increase in an amino acid sequence of collagen leads to decreasing and weakening collagen fibers in suppleness, strength, and elasticity. Since formation of this pathological crosslinking molecule is caused by blood glucose, its accumulation quantity is said to increase in a manner of depending on a dietary life habit (carbohydrate intake habit) of a host. Additionally, since saccharide matters being raw materials of pentosidine crosslinking molecules and of total glycation product crosslinking molecules including pentosidine crosslinking molecules are supplied in a transvascular manner, collagen being a supportive basis of tissues of a whole body is crosslinked, regardless of locations of the tissues, in accordance with a circulatory blood flow amount. That is, excessive intake of sugars can be said to lead to deteriorating uniformly quality of collagen to be a supportive basis of a body, regardless of whether in hard tissues or soft tissues.

Additionally, when pentosidine crosslinking molecules and total glycation product crosslinking molecules including pentosidine crosslinking molecules increase in collagen, pentosidine crosslinking molecules and total glycation product crosslinking molecules including pentosidine crosslinking molecules are glycation crosslinking molecules generated by Maillard reaction mediated by sugar, so that collagen contained in that living tissue takes on an appearance of a brittle chalk, and the collagen contained in that living tissue has its strength and calcification degree deteriorated. Although an aged one of a product generated by Maillard reaction mediated by sugar is said to be advanced glycation end-products (AGEs), pentosidine crosslinking molecules and total glycation product crosslinking molecules including pentosidine crosslinking molecules are AGEs and collagen of mammals which are kept in a state where a glucose level in blood is constantly high due to their dietary habits high in carbohydrate intake frequency contains a lot of pentosidine crosslinking molecules and total glycation product crosslinking molecules including pentosidine crosslinking molecules as compared with collagen of mammals which are at same ages (monthly ages) and whose dietary habits are well-balanced, and therefore, it can be said that a pathological crosslinking (aging crosslinking (AGEs crosslinking)) is advancing in collagen of mammals where a glucose level in blood is constantly high. The pentosidine crosslinking molecules and total glycation product crosslinking molecules including pentosidine crosslinking molecules, which has been generated, disturb a regular arrangement order of collagen fibers and make collagen have its flexibility impaired, and change it into a hard and brittle one, and simultaneously inhibit the collagen from being metabolized, and as a result, degrade quality of collagen constituting a whole body and cause tissues to deteriorate in suppleness. Since collagen is a crucial constituent of supportive tissues supporting not only all hard tissues such as bones and teeth but also all soft tissues including nerves and blood vessels, this deterioration of quality of collagen can be a factor of degrading quality of hard tissues/soft tissues of a whole body. For example, when glycation of bones deteriorates bone quality, the bones are embrittled, which causes osteoporosis, and besides, when quality of blood vessels deteriorates due to glycation of blood vessel walls, so-called arteriosclerosis, in which arteries are hardened, is generated, and thereby, a tissue disorder such as blood pressure elevation being caused is brought about. Similar matters hold true for "(1) diabetic retinopathy (if exacerbated, blindness)," "(2) diabetic nephropathy (if exacerbated, proceeding to kidney dialysis)," and "(3) diabetic neuropathy (if exacerbated, for example, gangrene of hand fingers and toe fingers due to blood circulation disorder)" called three major complications of diabetes, whose causes are said to be "(1) blood circulation disorder of retinae," "(2) capillary vessel disorder of kidney glomeruli being a blood filtration instrument," and "(3) blood flow disorder of distal parts of four limbs due to neuropathy of capillary vessels, and/or arteriosclerosis," respectively, and all of these are matters of which quality deterioration of blood vessels due to glycation of collagen constituting blood vessels can be said to be a major factor.

In addition, when advanced glycation end-products (AGEs) represented by pentosidine crosslinking molecules and total glycation product crosslinking molecules including pentosidine crosslinking molecules increase, AGEs bind to RAGEs (Receptor of age: receptor of AGEs) whose expression is found in a wide range of tissues such as blood-vessel system cells represented by vascular endothelial cells, and macrophages being an immunological cell, and as a result, an intracorporeal environment in which their signals are activated more easily is established. Thereby, NF-κB signals and so on being related to inflammation are promoted, and AGEs which are represented by pentosidine crosslinking molecules and total glycation product crosslinking molecules including pentosidine crosslinking molecules function on tissues excessively glycated as a chronic inflammation triggering factor, and thereby, induce chronic inflammation in a wide range of tissues. They are believed to be, by this mechanism, a factor of exacerbating chronic inflammatory diseases represented by inflammatory bowel diseases (IBDs), collagen diseases, and so on. As stated above, glycation of collagen leads to a factor of gradually undermining a living body in diverse directions, deteriorates quality of life (QOL) without being noticed, and can be a major factor of alienating living creatures from healthful longevity. It is reported that both pyridinoline crosslinking molecules and pentosidine crosslinking molecules in collagen contained in human living tissues increase due to aging (Shimizu, "Age-related nonenzymatic glycation modification of dentin collagen protein," Osaka University Knowledge Archive. 2015; Walters C. et al., Calcif. Tissue. Int. Vol. 35, 401-405, 1983).

A method according to the present invention, of using, as an index for evaluating a potential risk of developing a disease in a mammal and/or whether the mammal is potentially healthy, a quantitative value of pyridinoline content (ng/mg) of collagen contained in teeth of a mammal has process steps of (i)~(iii) below,
(i) a process step of obtaining a PYD being a quantitative value by quantifying pyridinoline content (ng/mg) of collagen contained in teeth of a mammal to be evaluated for a potential risk of developing a disease and/or whether the mammal is potentially healthy (evaluation subject pyridinoline content quantification process step),
(ii) a process step of obtaining an R-PYD being a reference quantitative value by quantifying pyridinoline content (ng/mg) of collagen contained in teeth of one or more mammals selected from a mammal that has not developed a disease, a mammal that has developed a disease, a healthy mammal, and a non-healthy mammal (reference pyridinoline content quantification process step),
(iii) a process step of comparing a PYD obtained in the evaluation subject pyridinoline content quantification process step (i) with an R-PYD obtained in the reference pyridinoline content quantification process step (ii) (PYD/R-PYD comparison process step).

Additionally, a method according to the present invention, of using, as an index for evaluating a potential risk of developing a disease in a mammal and/or whether the mammal is potentially healthy, a quantitative value of pentosidine content (ng/mg) of collagen contained in teeth of a mammal has process steps of (iv)~(vi) below,
(iv) a process step of obtaining a PEN being a quantitative value by quantifying pentosidine content (ng/mg) of collagen contained in teeth of a mammal to be evaluated for a potential risk of developing a disease and/or whether the mammal is potentially healthy (evaluation subject pentosidine content quantification process step),
(v) a process step of obtaining an R-PEN being a reference quantitative value by quantifying pentosidine content (ng/mg) of collagen contained in teeth of one or more mammals selected from a mammal that has not developed a disease, a mammal that has developed a disease, a healthy mammal, and a non-healthy mammal (reference pentosidine content quantification process step),
(vi) a process step of comparing a PEN obtained in the evaluation subject pentosidine content quantification process step (iv) with an R-PEN obtained in the reference pentosidine content quantification process step (v) (PEN/R-PEN comparison process step).

Additionally, a method according to the present invention, of using, as an index for evaluating a potential risk of developing a disease in a mammal and/or whether the mammal is potentially healthy, a quantitative value of total glycation product content (ng/mg: converted to pentosidine content) of collagen contained in teeth of a mammal has process steps of (vii)~(ix) below,
(vii) a process step of obtaining a T-GLYC being a quantitative value by quantifying total glycation product content (ng/mg: converted to pentosidine content) of collagen contained in teeth of a mammal to be evaluated for a potential risk of developing a disease and/or whether the mammal is potentially healthy (evaluation subject total glycation product content quantification process step),
(viii) a process step of obtaining an R-T-GLYC being a reference quantitative value by quantifying total glycation product content (ng/mg: converted to pentosidine content) of collagen contained in teeth of one or more mammals selected from a mammal that has not developed a disease, a mammal that has developed a disease, a healthy mammal, and a non-healthy mammal (reference total glycation product content quantification process step),
(ix) a process step of comparing a T-GLYC obtained in the evaluation subject total glycation product content quantification process step (vii) with an R-T-GLYC obtained in the reference total glycation product content quantification process step (viii) (T-GLYC/R-T-GLYC comparison process step).

Additionally, a method according to the present invention, of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of pentosidine content thereof has process steps of (x)~(xvi) below,
(x) a process step of obtaining a PYD being a quantitative value by quantifying pyridinoline content (ng/mg) of collagen contained in teeth of a mammal to be evaluated for a potential risk of developing a disease and/or whether the mammal is potentially healthy (evaluation subject pyridinoline content quantification process step),
(xi) a process step of obtaining a PEN being a quantitative value by quantifying pentosidine content (ng/mg) of collagen contained in teeth of said mammal to be evaluated (evaluation subject pentosidine content quantification process step),
(xii) a process step of calculating a PpP (pyridinoline content (ng/mg)/ pentosidine content (ng/mg)) being a value of a weight ratio, from a PYD obtained in the evaluation subject pyridinoline content quantification process step (x) and a PEN obtained in the evaluation subject pentosidine content quantification process step (xi) (evaluation subject weight ratio PpP calculation process step),
(xiii) a process step of obtaining an R-PYD being a reference quantitative value by quantifying pyridinoline content (ng/mg) of collagen contained in teeth of one or more mammals selected from a mammal that has not developed a disease, a mammal that has developed a disease, a healthy mammal, and a non-healthy mammal (reference pyridinoline content quantification process step), (xiv) a process step of obtaining an R-PEN being a reference quantitative value by quantifying pentosidine content (ng/mg) of collagen contained in teeth of said one or more mammals (reference pentosidine content quantification process step),
(xv) a process step of calculating an R-PpP (pyridinoline content (ng/mg)/ pentosidine content (ng/mg)) being a reference value of a weight ratio, from an R-PYD obtained in the reference pyridinoline content quantification process step (xiii) and an R-PEN obtained in the reference pentosidine content quantification process step (xiv) (reference weight ratio R-PpP calculation process step),
(xvi) a process step of comparing a PpP calculated in the evaluation subject weight ratio PpP calculation process step (xii) with an R-PpP calculated in the reference weight ratio R-PpP calculation process step (xv) (weight ratio PpP/R-PpP comparison process step).

Additionally, a method according to the present invention, of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of total glycation product content thereof has process steps of (xvii)~(xxiii) below,
(xvii) a process step of obtaining a PYD being a quantitative value by quantifying pyridinoline content (ng/mg) of collagen contained in teeth of a mammal to be evaluated for a potential risk of developing a disease and/or whether the mammal is potentially healthy (evaluation subject pyridinoline content quantification process step),
(xviii) a process step of obtaining a T-GLYC being a quantitative value by quantifying total glycation product content (ng/mg: converted to pentosidine content) of collagen contained in teeth of said mammal to be evaluated (evaluation subject total glycation product content quantification process step),
(xix) a process step of calculating a PpG (pyridinoline content (ng/mg)/ total glycation product content (ng/mg: converted to pentosidine content)) being a value of a weight ratio, from a PYD obtained in the evaluation subject pyridinoline content quantification process step (xvii) and a T-GLYC obtained in the evaluation subject total glycation product content quantification process step (xviii) (evaluation subject weight ratio PpG calculation process step),
(xx) a process step of obtaining an R-PYD being a reference quantitative value by quantifying pyridinoline content (ng/mg) of collagen contained in teeth of one or more mammals selected from a mammal that has not developed a disease, a mammal that has developed a disease, a healthy mammal, and a non-healthy mammal (reference pyridinoline content quantification process step),
(xxi) a process step of obtaining an R-T-GLYC being a reference quantitative value by quantifying total glycation product content (ng/mg: converted to pentosidine content) of collagen contained in teeth of said one or more mammals (reference total glycation product content quantification process step),
(xxii) a process step of calculating an R-PpG (pyridinoline content (ng/mg)/ total glycation product content (ng/mg: converted to pentosidine content)) being a reference value of a weight ratio, from an R-PYD obtained in the reference pyridinoline content quantification process step (xx) and an R-T-GLYC obtained in the reference total glycation product content quantification process step (xxi) (reference weight ratio R-PpG calculation process step),
(xxiii) a process step of comparing a PpG calculated in the evaluation subject weight ratio PpG calculation process step (xix) with an R-PpG calculated in the reference weight ratio R-PpG calculation process step (xxii) (weight ratio PpG/R-PpG comparison process step).

The "evaluation subject pyridinoline content quantification process step (i)" in a method according to the present invention, of using, as an index for evaluating a potential risk of developing a disease in a mammal and/or whether the mammal is potentially healthy, a quantitative value of pyridinoline content (ng/mg) of collagen contained in teeth of a mammal, the "evaluation subject pyridinoline content quantification process step (x)" in a method according to the present invention, of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of pentosidine content thereof, and the "evaluation subject pyridinoline content quantification process step (xvii)" in a method according to the present invention, of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of total glycation product content thereof are identical process steps.

Additionally, the "reference pyridinoline content quantification process step (ii)" in a method according to the present invention, of using, as an index for evaluating a potential risk of developing a disease in a mammal and/or whether the mammal is potentially healthy, a quantitative value of pyridinoline content (ng/mg) of collagen contained in teeth of a mammal, the "reference pyridinoline content quantification process step (xiii)" in a method according to the present invention, of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of pentosidine content thereof, and the "reference pyridinoline content quantification process step (xx)" in a method according to the present invention, of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of total glycation product content thereof are identical process steps.

Additionally, the "evaluation subject pentosidine content quantification process step (iv)" in a method according to the present invention, of using, as an index for evaluating a potential risk of developing a disease in a mammal and/or whether the mammal is potentially healthy, a quantitative value of pentosidine content (ng/mg) of collagen contained in teeth of a mammal, and the "evaluation subject pentosidine content quantification process step (xi)" in a method according to the present invention, of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of pentosidine content thereof are identical process steps.

Additionally, the "reference pentosidine content quantification process step (v)" in a method according to the present invention, of using, as an index for evaluating a potential risk of developing a disease in a mammal and/or whether the mammal is potentially healthy, a quantitative value of pentosidine content (ng/mg) of collagen contained in teeth of a mammal, and the "reference pentosidine content quantification process step (xiv)" in a method according to the present invention, of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of pentosidine content thereof are identical process steps.

Additionally, the "evaluation subject total glycation product content quantification process step (vii)" in a method according to the present invention, of using, as an index for evaluating a potential risk of developing a disease in a mammal and/or whether the mammal is potentially healthy, a quantitative value of total glycation product content (ng/mg: converted to pentosidine content) of collagen contained in teeth of a mammal, and the "evaluation subject total glycation product content quantification process step (xviii)" in a method according to the present invention, of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of total glycation product content thereof are identical process steps.

Additionally, the "reference total glycation product content quantification process step (viii)" in a method according to the present invention, of using, as an index for evaluating a potential risk of developing a disease in a mammal and/or whether the mammal is potentially healthy, a quantitative value of total glycation product content (ng/mg: converted to pentosidine content) of collagen contained in teeth of a mammal, and the "reference total glycation product content quantification process step (xxi)" in a method according to the present invention, of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of total glycation product content thereof are identical process steps.

Although preparation of respective samples of pyridinoline, pentosidine, total glycation products provided for quantification of pyridinoline content (ng/mg) in "evaluation subject pyridinoline content quantification process step" and "reference pyridinoline content quantification process step," quantification of pentosidine content (ng/mg) in "evaluation subject pentosidine content quantification process step" and "reference pentosidine content quantification process step," as well as quantification of total glycation product content (ng/mg: converted to pentosidine content) in "evaluation subject total glycation product content quantification process step" and "reference total glycation product content quantification process step" can be carried out by using a conventional preparation means publicly known in this field, it can be carried out, for example, by and large as follows.
(1) Measure dried collagen powder produced by means of a method of producing a collagen-rich organic composition matter according to the present invention, take it into a test tube, and swell it by means of a small amount of 6N hydrochloric acid under deaeration.
(2) After making said dried collagen have 6N hydrochloric acid added and deaerating it, seal it.
(3) Hydrolyze it by giving it heating treatment at 110°C for one whole day and night.
(4) Cool a sample to room temperature.
(5) Obtain a collagen hydrolysate by drying, while warming, it under reduced pressure so as to remove surplus hydrochloric acid.
(6) Solubilize the collagen hydrolysate obtained, by means of 10% methanol aqueous solution.
(7) Centrifuge it at 12000 rpm under room temperature for 5 minutes and make a supernatant a sample.
(8) Obtain an extraction rate and a calibration curve by adding a known amount of a standard substance to the sample, by which collagen contents can be corrected as appropriate.

Additionally, although a quantification method of pyridinoline content (ng/mg) in "evaluation subject pyridinoline content quantification process step" and "reference pyridinoline content quantification process step," a quantification method of pentosidine content (ng/mg) in "evaluation subject pentosidine content quantification process step" and "reference pentosidine content quantification process step," as well as a quantification method of total glycation product content (ng/mg: converted to pentosidine content) in "evaluation subject total glycation product content quantification process step" and "reference total glycation product content quantification process step" are not limited in particular as long as those quantifications can be carried out, a high-performance liquid chromatograph with a fluorescence detector (HPLC-Flu) using heptafluorobutyric acid and formic acid as an ion-pairing reagent can be adopted, for example, as such quantification methods.

Although this quantification method using a high-performance liquid chromatograph with a fluorescence detector (HPLC-Flu) using heptafluorobutyric acid and formic acid as an ion-pairing reagent can be carried out by using a conventional quantification means publicly known in this field, it can be carried out, for example, by and large as follows.
(1) Use, as a mobile phase, a ultrapure water (mobile phase A) containing 0.05% heptafluorobutyric acid, and a methanol/ethanol 1:1 mixture solution (mobile phase B) containing 0.05% formic acid, to make liquid delivery in a gradient setting and carry out detection.
(2) As for a specific liquid delivery condition, initiate liquid delivery with a detection column temperature being 40°C and a flow rate being 0.5 mL/min and in a proportion of mobile phase A 90% and mobile phase B 10%, and make it for 0.5 minutes.
(3) Thereafter, after increasing the mobile phase B to 22% by spending 1 minute and applying a concentration gradient, make liquid delivery for 7.5 minutes as it is.
(4) Followingly, after increasing the mobile phase B to 80% by spending 1 minute, make liquid delivery for 1 minute, further.
(5) Finally, after making liquid delivery in a proportion of mobile phase A 10% and mobile phase B 90%, make equilibration for 4 minutes in a proportion of mobile phase A 90% and mobile phase B 10%.
(6) Carry out (1)~(5) above by spending 15 minutes in total for one sample. Use Cadenza CD-C18 with its length 150 mm and its inner diameter 3 mm for a separation column, and use a fluorescent detector for detection. Monitor such for first-half 8 minutes at 295 nm in excitation wavelength and 395 nm in fluorescence wavelength, and monitor it for second-half 7 minutes at 325 nm in excitation wavelength and 385 nm in fluorescence wavelength. By monitoring those, pyridinoline contents are detected at a retention time of about 6.5 minutes or around, and pentosidine contents are detected at a retention time of about 9.1 minutes or around.

Quantification of pyridinoline content (ng/mg) in "evaluation subject pyridinoline content quantification process step" and "reference pyridinoline content quantification process step," quantification of pentosidine content (ng/mg) in "evaluation subject pentosidine content quantification process step" and "reference pentosidine content quantification process step," as well as quantification of total glycation product content (ng/mg: converted to pentosidine content) in "evaluation subject total glycation product content quantification process step" and "reference total glycation product content quantification process step" can be carried out on teeth extracted from a mammal to be evaluated for a potential risk of developing a disease and/or whether the mammal is potentially healthy, a mammal that has not developed a disease, a mammal that has developed a disease, a healthy mammal, and a non-healthy mammal, and besides, can be carried out even in modes such as cutting, contact, and non-contact, for example.

Additionally, it is allowable that any of a mammal to be evaluated for a potential risk of developing a disease and/or whether the mammal is potentially healthy, a mammal that has not developed a disease, a mammal that has developed a disease, a healthy mammal, and a non-healthy mammal is an identical individual.

It is allowable that a method according to the present invention, of using, as an index for evaluating a potential risk of developing a disease in a mammal and/or whether the mammal is potentially healthy, a quantitative value of pyridinoline content (ng/mg) of collagen contained in teeth of a mammal has not only the above-mentioned "evaluation subject pyridinoline content quantification process step (i)," "reference pyridinoline content quantification process step (ii)," and "PYD/R-PYD comparison process step (iii)," but also, to an extent that features of the present invention are not compromised, another process step.

Additionally, it is allowable that a method according to the present invention, of using, as an index for evaluating a potential risk of developing a disease in a mammal and/or whether the mammal is potentially healthy, a quantitative value of pentosidine content (ng/mg) of collagen contained in teeth of a mammal has not only the above-mentioned "evaluation subject pentosidine content quantification process step (iv)," "reference pentosidine content quantification process step (v)," and "PEN/R-PEN comparison process step (vi)," but also, to an extent that features of the present invention are not compromised, another process step.

Additionally, it is allowable that a method according to the present invention, of using, as an index for evaluating a potential risk of developing a disease in a mammal and/or whether the mammal is potentially healthy, a quantitative value of total glycation product content (ng/mg: converted to pentosidine content) of collagen contained in teeth of a mammal has not only the above-mentioned "evaluation subject total glycation product content quantification process step (vii)," "reference total glycation product content quantification process step (viii)," and "T-GLYC/R-T-GLYC comparison process step (ix)," but also, to an extent that features of the present invention are not compromised, another process step.

Additionally, it is allowable that a method according to the present invention, of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of pentosidine content thereof has not only the above-mentioned "evaluation subject pyridinoline content quantification process step (x)," "evaluation subject pentosidine content quantification process step (xi)," "evaluation subject weight ratio PpP calculation process step (xii)," "reference pyridinoline content quantification process step (xiii)," "reference pentosidine content quantification process step (xiv)," "reference weight ratio R-PpP calculation process step (xv)," and "weight ratio PpP/R-PpP comparison process step (xvi)," but also, to an extent that features of the present invention are not compromised, another process step.

Additionally, it is allowable that a method according to the present invention, of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of total glycation product content thereof has not only the above-mentioned "evaluation subject pyridinoline content quantification process step (xvii)," "evaluation subject total glycation product content quantification process step (xviii)," "evaluation subject weight ratio PpG calculation process step (xix)," "reference pyridinoline content quantification process step (xx)," "reference total glycation product content quantification process step (xxi)," "reference weight ratio R-PpG calculation process step (xxii)," and "weight ratio PpG/R-PpG comparison process step (xxiii)," but also, to an extent that features of the present invention are not compromised, another process step.

A reason why limitation to "teeth" is placed in a method according to the present invention, of using, as an index for evaluating a potential risk of developing a disease in a mammal and/or whether the mammal is potentially healthy, a quantitative value of pyridinoline content (ng/mg) of collagen contained in teeth of a mammal, a method according to the present invention, of using, as an index for evaluating a potential risk of developing a disease in a mammal and/or whether the mammal is potentially healthy, a quantitative value of pentosidine content (ng/mg) of collagen contained in teeth of a mammal, a method according to the present invention, of using, as an index for evaluating a potential risk of developing a disease in a mammal and/or whether the mammal is potentially healthy, a quantitative value of total glycation product content (ng/mg: converted to pentosidine content) of collagen contained in teeth of a mammal, a method according to the present invention, of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of pentosidine content thereof, as well as in a method according to the present invention, of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of total glycation product content thereof is that all of pyridinoline crosslinking, pentosidine crosslinking, and total glycation product crosslinking in type I collagen are a crosslinking reaction which is accelerated by a factor such as physical internal heat based on body temperature and sugar supplied in a transvascular manner, and a crosslinking reaction which is caused not locally but in accordance with a circulatory blood flow amount, and that among them, it is collagen contained in teeth (particularly, dentin) that is considered to reflect most accurately accumulation of pyridinoline crosslinking molecules, pentosidine crosslinking molecules, and total glycation product crosslinking molecules including pentosidine crosslinking molecules. That is because, since teeth are sole tissues not being incorporated in metabolism cycles in a living body and are neither absorbed nor decomposed once formed, pyridinoline crosslinking molecules, pentosidine crosslinking molecules, and total glycation product crosslinking molecules including pentosidine crosslinking molecules are accumulated in collagen contained in teeth (particularly, dentin).

Furthermore, collagen of teeth (particularly, dentin) is insoluble as compared with that of bones. Pepsin digestion under a condition of 0.01N hydrochloric acid, 4°C, 72 hours of treatment time solubilizes approximately 35% of collagen as for adult bovines' bones, but only solubilizes 5.6% of collagen as for dentin of adult bovines' teeth. Additionally, insoluble collagen of skins, Achilles tendons, and so on is swollen 4~8 times in volume under a condition of pH2, and insoluble collagen of adult bovines' bones is swollen 1.2 times in volume thereunder, whereas insoluble collagen of adult bovines' dentin is not swollen at all thereunder (Yutaka Nagai and Daizaburo Fujimoto (eds.), Collagen Experimental Methods, Kodansha Scientific, pp. 21-22). Additionally, dentin collagen of teeth is highest in content rate of pyridinoline crosslinking molecules in a living body. Accordingly, it is with reason to evaluate, by making a PYD (ng/mg) being a quantitative value of pyridinoline content of collagen contained in teeth of a mammal, a PEN (ng/mg) being a quantitative value of pentosidine content of collagen contained in teeth of a mammal, a T-GLYC (ng/mg: converted to pentosidine content) being a quantitative value of total glycation product content of collagen contained in teeth of a mammal, a PpP (pyridinoline content (ng/mg)/ pentosidine content (ng/mg)) being a value of a weight ratio calculated from a PYD (ng/mg) being a quantitative value of pyridinoline content of collagen contained in teeth of a mammal and a PEN (ng/mg) being a quantitative value of pentosidine content thereof, as well as a PpG (pyridinoline content (ng/mg)/ total glycation product content (ng/mg: converted to pentosidine content)) being a value of a weight ratio calculated from a PYD (ng/mg) being a quantitative value of pyridinoline content of collagen contained in teeth of a mammal and a T-GLYC (ng/mg: converted to pentosidine content) being a quantitative value of total glycation product content thereof be indices, a potential risk of developing a disease in a mammal and/or whether the mammal is potentially healthy.

Meanwhile, grounds for bones having been excluded shall be explained. Although as an index of osteoporosis, numerical values of type I collagen C-terminus telopeptide (ICTP) in blood (serum) and NTx (type I collagen crosslinking N-telopeptide) in urine are pervasively utilized, these are substances which are, as collagen decomposition products due to matrix metalloprotease (MMPs) activity, released into blood/urine, and are substances containing pyridinoline crosslinking molecules. From this, it can be made out that pyridinoline crosslinking molecules, pentosidine crosslinking molecules, and total glycation product crosslinking molecules including pentosidine crosslinking molecules, contained in bone-contained collagen are partially given clearance through metabolism, and in addition to such, it becomes very difficult to purify collagen into one high in purity since in a case of using extracted bones, defatting action is not easy because bones are an organ containing a large amount of lipid derived from yellow bone marrow.

A method according to the present invention, of using, as an index for evaluating a potential risk of developing a disease in a mammal, a PYD being a quantitative value of pyridinoline content of collagen contained in teeth of a mammal, a method according to the present invention, of using, as an index for evaluating a potential risk of developing a disease in a mammal, a PEN being a quantitative value of pentosidine content of collagen contained in teeth of a mammal, a method according to the present invention, of using, as an index for evaluating a potential risk of developing a disease in a mammal, a T-GLYC being a quantitative value of total glycation product content of collagen contained in teeth of a mammal, a method according to the present invention, of using, as an index for evaluating a potential risk of developing a disease in a mammal, a value of a PpP (pyridinoline content/ pentosidine content) being a value of a weight ratio calculated from a PYD being a quantitative value of pyridinoline content of collagen contained in teeth of a mammal and a PEN being a quantitative value of pentosidine content thereof, a method according to the present invention, of using, as an index for evaluating a potential risk of developing a disease in a mammal, a value of a PpG (pyridinoline content/ total glycation product content) being a value of a weight ratio calculated from a PYD being a quantitative value of pyridinoline content of collagen contained in teeth of a mammal and a T-GLYC being a quantitative value of total glycation product content thereof, a method according to the present invention, of using, as an index for evaluating whether a mammal is potentially healthy, a PYD being a quantitative value of pyridinoline content of collagen contained in teeth of a mammal, a method according to the present invention, of using, as an index for evaluating whether a mammal is potentially healthy, a PEN being a quantitative value of pentosidine content of collagen contained in teeth of a mammal, a method according to the present invention, of using, as an index for evaluating whether a mammal is potentially healthy, a T-GLYC being a quantitative value of total glycation product content of collagen contained in teeth of a mammal, a method according to the present invention, of using, as an index for evaluating whether a mammal is potentially healthy, a value of a PpP (pyridinoline content/ pentosidine content) being a value of a weight ratio calculated from a PYD being a quantitative value of pyridinoline content of collagen contained in teeth of a mammal and a PEN being a quantitative value of pentosidine content thereof, as well as a method according to the present invention, of using, as an index for evaluating whether a mammal is potentially healthy, a value of a PpG (pyridinoline content/ total glycation product content) being a value of a weight ratio calculated from a PYD being a quantitative value of pyridinoline content of collagen contained in teeth of a mammal and a T-GLYC being a quantitative value of total glycation product content thereof may be independent of, and may be related to each other.

A method according to the present invention, of using, as an index for evaluating a potential risk of developing a disease in a mammal and/or whether the mammal is potentially healthy, a quantitative value of pyridinoline content (ng/mg) of collagen contained in teeth of a mammal, a method according to the present invention, of using, as an index for evaluating a potential risk of developing a disease in a mammal and/or whether the mammal is potentially healthy, a quantitative value of pentosidine content (ng/mg) of collagen contained in teeth of a mammal, a method according to the present invention, of using, as an index for evaluating a potential risk of developing a disease in a mammal and/or whether the mammal is potentially healthy, a quantitative value of total glycation product content (ng/mg: converted to pentosidine content) of collagen contained in teeth of a mammal, a method according to the present invention, of using, as an index for evaluating a potential risk of developing a disease in a mammal and/or whether the mammal is potentially healthy, a value of a weight ratio (pyridinoline content (ng/mg)/ pentosidine content (ng/mg)) calculated from a quantitative value of pyridinoline content (ng/mg) of collagen contained in teeth of a mammal and a quantitative value of pentosidine content (ng/mg) thereof, as well as a method according to the present invention, of using, as an index for evaluating a potential risk of developing a disease in a mammal and/or whether the mammal is potentially healthy, a value of a weight ratio (pyridinoline content (ng/mg)/ total glycation product content (ng/mg: converted to pentosidine content)) calculated from a quantitative value of pyridinoline content (ng/mg) of collagen contained in teeth of a mammal and a quantitative value of total glycation product content (ng/mg: converted to pentosidine content) thereof are described on a basis of embodiment examples, below. Technical scope of the present invention is not limited to embodiments shown by these embodiment examples.

### [Embodiment Example]

### <Embodiment Example 1> Measurement of pyridinoline quantity, pentosidine quantity, and total glycation product quantity in collagen derived from teeth (mainly, dentin) of human non-diabetic patients, human diabetic patients, and bovines

Pyridinoline quantity (ng/mg), pentosidine quantity (ng/mg), and total glycation product quantity (ng/mg: converted to pentosidine content) in collagen derived from teeth (mainly, dentin) of human non-diabetic patients 14 cases, human diabetic patients 4 cases, and bovines ((Specified Risk Materials; SRM) of bovines) 19 cases were measured by means of procedures below.

### 1. Preparation of collagen powder

[1-1] By means of a pulverizing machine, a hard tissue was pulverized to a degree of 1 mm in particle size.
[1-2] In a 1% sodium carbonate aqueous solution, agitation treatment was carried out for several hours under a condition of 70°C and soft tissues attached were removed.
[1-3] Demineralization treatment was carried out. To go into details, after demineralization treatment was, by demineralizing solution containing 1M phosphoric acid and ethanol being used, carried out for several hours while depressurization was being carried out, demineralization treatment was, by said demineralizing solution diluted in a few multiple being substituted, carried out for several hours while depressurization was being carried out. Followingly, demineralization was, by the above-mentioned demineralizing solution diluted fivefold being used and by a shake being given for about one whole day and night under a room-temperature condition, carried out completely. Thereafter, a wash was, by 5% ethanol being used, made several times under a room-temperature condition.
[1-4] The hard tissue after being demineralized was finely powdered by being moved into a mortar, and being rubbed and crushed in 70% ethanol.
[1-5] It was agitated in 100% ethanol, was washed, and defatted, dewatered.
[1-6] Ethanol left in collagen was completely removed by drying, and dried collagen powder was obtained.

### 2. Preparation of an analytical sample

[2-1] The dried collagen powder obtained in [1-6] was hydrolyzed. To go into details, after the dried collagen powder obtained was measured and taken into a test tube, and was fully swollen by means of a small amount of 6N hydrochloric acid under deaeration, a suitable amount of 6N hydrochloric acid was added to the dried collagen and the test tube was welded under deaeration to be sealed. After hydrolysis was carried out by the test tube sealed being given overnight heating treatment under a condition of 110°C for one whole day and night, the test tube was cooled to room temperature and severed, and surplus hydrochloric acid was removed by drying under reduced pressure while being warmed, and a collagen hydrolysate was obtained.

[2-2] The collagen hydrolysate obtained was solubilized by means of 10% methanol aqueous solution, and was centrifuged for 5 minutes at 12000 rpm under room temperature, and a supernatant was made to be a sample.

[2-3] By a known amount of a standard substance being added to the sample, an extraction rate and a calibration curve were obtained so that collagen contents were corrected as appropriate.

3. Quantification of pyridinoline content, pentosidine content, and total glycation product content, by means of a high-performance liquid chromatograph with a fluorescence detector (HPLC-Flu) using heptafluorobutyric acid and formic acid as an ion-pairing reagent, and calculation of a weight ratio of those

By a ultrapure water (mobile phase A) containing 0.05% heptafluorobutyric acid, and a methanol/ethanol 1:1 mixture solution (mobile phase B) containing 0.05% formic acid being used as a mobile phase, liquid delivery was made in a gradient setting and detection was made. Specifically, liquid delivery was initiated with a detection column temperature being 40°C and a flow rate being 0.5 mL/min and in a proportion of mobile phase A 90% and mobile phase B 10%, and was made for 0.5 minutes. Thereafter, after the mobile phase B was increased to 22% by spending 1 minute so that a concentration gradient was applied, liquid delivery was made for 7.5 minutes as it was. Followingly, after the mobile phase B was increased to 80% by spending 1 minute, liquid delivery was further made for 1 minute. Finally, after liquid delivery was made in a proportion of mobile phase A 10% and mobile phase B 90%, subsequently the mobile phase B was decreased to 10%, and equilibration was made for 4 minutes in a proportion of mobile phase A 90% and mobile phase B 10%. Followingly, detection was made by spending 15 minutes in total for one sample. Cadenza CD-C18 with its length 150 mm and its inner diameter 3 mm was used for a separation column and a fluorescent detector was used for detection. Such was monitored for first-half 8 minutes at 295 nm in excitation wavelength and 395 nm in fluorescence wavelength, and was monitored for second-half 7 minutes at 325 nm in excitation wavelength and 385 nm in fluorescence wavelength. By such being monitored, pyridinoline contents were detected at a retention time of about 6.5 minutes or around, whereas pentosidine contents and total glycation product contents were detected at a retention time of about 9.1 minutes or around. From pyridinoline contents and total glycation product contents obtained, a weight ratio of those (pyridinoline content (ng/mg)/ total glycation product content (ng/mg: converted to pentosidine content)) was obtained.

As described above, a distribution diagram according to monthly ages of teeth, of a PYD being a quantitative value of pyridinoline content (ng/mg) of collagen contained in teeth of human non-diabetic patients 14 cases, human diabetic patients 4 cases, and bovines 19 cases is shown in Fig.1, a distribution diagram according to monthly ages of teeth, of a PEN being a quantitative value of pentosidine content (ng/mg) of collagen contained in teeth of human non-diabetic patients 14 cases, human diabetic patients 4 cases, and bovines 19 cases is shown in Fig.2, a distribution diagram according to monthly ages of teeth, of a T-GLYC being a quantitative value of total glycation product content (ng/mg: converted to pentosidine content) of collagen contained in teeth of human non-diabetic patients 14 cases, human diabetic patients 4 cases, and bovines 19 cases is shown in Fig.3, a distribution diagram according to monthly ages of teeth, of a PpP (pyridinoline content (ng/mg)/ pentosidine content (ng/mg)) being a value of a weight ratio calculated from a PYD being a quantitative value of pyridinoline content (ng/mg) and a PEN being a quantitative value of pentosidine content (ng/mg), of collagen contained in teeth of human non-diabetic patients 14 cases, human diabetic patients 4 cases, and bovines 19 cases is shown in Fig.4, a distribution diagram according to monthly ages of teeth, of a PpG (pyridinoline content (ng/mg)/ total glycation product content (ng/mg: converted to pentosidine content)) being a value of a weight ratio calculated from a PYD being a quantitative value of pyridinoline content (ng/mg) and a T-GLYC being a quantitative value of total glycation product content (ng/mg: converted to pentosidine content), of collagen contained in teeth of human non-diabetic patients 14 cases, human diabetic patients 4 cases, and bovines 19 cases is shown in Fig.5, for each. In Fig.1, a broken line represents an approximation curve (linear approximation) based on PYDs of human non-diabetic patients 14 cases, which correspond to an R-PYD, and in Fig.2, a broken line represents an approximation curve (linear approximation) based on PENs of human non-diabetic patients 14 cases, which correspond to an R-PEN, and in Fig.3, a broken line represents an approximation curve (linear approximation) based on T-GLYCs of human non-diabetic patients 14 cases, which correspond to an R-T-GLYC, and in Fig.4, a broken line represents an approximation curve (linear approximation) based on PpPs of human non-diabetic patients 14 cases, which correspond to an R-PpP, and in Fig.5, a broken line represents an approximation curve (linear approximation) based on PpGs of human non-diabetic patients 14 cases, which correspond to an R-PpG.

Although permanent teeth are made to be a measurement target with regard to human teeth, it can be thought in consideration of a formation period of permanent tooth dentin that dentin formation is initiated in a tooth germ inside a jawbone at an age around 10 years old on average and along therewith glycation crosslinking formation is also initiated (Chronology of Deciduous and Permanent Dentition in Japanese Children II, The Japanese Journal of Pediatric Dentistry 57(3), 363-373, 2019, 363). Meanwhile, since a similar matter can be said 18 months or more after birth on average with regard to lower jaw teeth of bovine SRM as well, Table 1 below is used for converting a yearly age into a monthly age with regard to specified teeth of humans and bovine SRM.

**[Table 1]**

| **Teeth eruption monthly age** | | | |
|---|---|---|---|
| | Tooth type | Male | Female |
| Human upper jaw | 1 | 86 | 83 |
| | 2 | 100 | 95 |
| | 3 | 132 | 123 |
| | 4 | 124 | 120 |
| | 5 | 141 | 138 |
| | 6 | 87 | 85 |
| | 7 | 159 | 156 |
| | 8 | 204 | 204 |
| Human lower jaw | 1 | 75 | 72 |
| | 2 | 87 | 84 |
| | 3 | 123 | 114 |
| | 4 | 125 | 121 |
| | 5 | 140 | 140 |
| | 6 | 80 | 75 |
| | 7 | 151 | 150 |
| | 8 | 204 | 204 |
| Bovine SRM | Lower 1 | 18 | 18 |

Additionally, a PYD being a quantitative value of pyridinoline content (ng/mg) of collagen contained in teeth of human non-diabetic patients 14 cases, a PEN being a quantitative value of pentosidine content (ng/mg) thereof, a T-GLYC being a quantitative value of total glycation product content (ng/mg: converted to pentosidine content) thereof, which were determined through quantification by means of a high-performance liquid chromatograph with a fluorescence detector (HPLC-Flu) using heptafluorobutyric acid and formic acid as an ion-pairing reagent, as well as a PpP (pyridinoline content (ng/mg)/ pentosidine content (ng/mg)) being a value of a weight ratio calculated from a PYD being a quantitative value of pyridinoline content (ng/mg) and a PEN being a quantitative value of pentosidine content (ng/mg), a PpG (pyridinoline content (ng/mg)/ total glycation product content (ng/mg: converted to pentosidine content)) being a value of a weight ratio calculated from a PYD being a quantitative value of pyridinoline content (ng/mg) and a T-GLYC being a quantitative value of total glycation product content (ng/mg: converted to pentosidine content) are shown in Table 2 below.

**[Table 2]**

| | Human Non-diabetic | | | | | | |
|---|---|---|---|---|---|---|---|
| Monthly age | 24 | 176 | 176 | 175 | 175 | 96 | 180 |
| PYD | 391.7395 | 432.1056 | 338.7585 | 423.5153 | 442.5367 | 409.229 | 441.4954 |
| PEN | 0.31369 | 0.480136 | 0.319634 | 0.512231 | 0.598219 | 0.410702 | 0.620344 |
| T-GLYC | 1.668493 | 4.796875 | 4.055365 | 4.945752 | 5.382466 | 2.186311 | 3.327377 |
| PpP | 1248.809 | 899.9658 | 1059.834 | 826.8061 | 739.7574 | 996.4133 | 711.695 |
| PpG | 234.7864 | 90.08065 | 83.53342 | 85.63214 | 82.21821 | 187.1779 | 132.6857 |

| Monthly age | 192 | 300 | 324 | 516 | 636 | 876 | 844 |
|---|---|---|---|---|---|---|---|
| PYD | 463.8142 | 408.0093 | 455.653 | 381.3666 | 326.0682 | 312.4233 | 455.3794 |
| PEN | 0.60686 | 0.681059 | 0.818682 | 1.025046 | 0.865236 | 0.932765 | 0.709146 |
| T-GLYC | 3.644623 | 3.713026 | 4.455984 | 5.126387 | 4.817169 | 4.204468 | 3.740644 |
| PpP | 764.2859 | 599.0811 | 556.5693 | 372.0482 | 376.8546 | 334.9432 | 642.1518 |
| PpG | 127.2599 | 109.8859 | 102.2564 | 74.39287 | 67.68876 | 74.30746 | 121.7382 |

Next, a PYD being a quantitative value of pyridinoline content (ng/mg) of collagen contained in teeth of human diabetic patients 4 cases, a PEN being a quantitative value of pentosidine content (ng/mg) thereof, a T-GLYC being a quantitative value of total glycation product content (ng/mg: converted to pentosidine content) thereof, which were determined through quantification by means of a high-performance liquid chromatograph with a fluorescence detector (HPLC-Flu) using heptafluorobutyric acid and formic acid as an ion-pairing reagent, as well as a PpP (pyridinoline content (ng/mg)/ pentosidine content (ng/mg)) being a value of a weight ratio calculated from a PYD being a quantitative value of pyridinoline content (ng/mg) and a PEN being a quantitative value of pentosidine content (ng/mg), a PpG (pyridinoline content (ng/mg)/ total glycation product content (ng/mg: converted to pentosidine content)) being a value of a weight ratio calculated from a PYD being a quantitative value of pyridinoline content (ng/mg) and a T-GLYC being a quantitative value of total glycation product content (ng/mg: converted to pentosidine content) are shown in Table 3 below.

**[Table 3]**

| | Human Diabetic | | | |
|---|---|---|---|---|
| Monthly age | 336 | 456 | 876 | 864 |
| PYD | 446.7094 | 376.2082 | 313.4794 | 386.1348 |
| PEN | 1.308556 | 1.221355 | 1.772419 | 1.383665 |
| T-GLYC | 6.127007 | 7.585624 | 9.149883 | 7.47555 |
| PpP | 341.3758 | 308.0252 | 176.8653 | 279.0667 |
| PpG | 72.90826 | 49.59489 | 34.26048 | 51.65303 |

Next, a PYD being a quantitative value of pyridinoline content (ng/mg) of collagen contained in teeth of bovines 19 cases, a PEN being a quantitative value of pentosidine content (ng/mg) thereof, a T-GLYC being a quantitative value of total glycation product content (ng/mg: converted to pentosidine content) thereof, which were determined through quantification by means of a high-performance liquid chromatograph with a fluorescence detector (HPLC-Flu) using heptafluorobutyric acid and formic acid as an ion-pairing reagent, as well as a PpP (pyridinoline content (ng/mg)/ pentosidine content (ng/mg)) being a value of a weight ratio calculated from a PYD being a quantitative value of pyridinoline content (ng/mg) and a PEN being a quantitative value of pentosidine content (ng/mg), a PpG (pyridinoline content (ng/mg)/ total glycation product content (ng/mg: converted to pentosidine content)) being a value of a weight ratio calculated from a PYD being a quantitative value of pyridinoline content (ng/mg) and a T-GLYC being a quantitative value of total glycation product content (ng/mg: converted to pentosidine content) are shown in Table 4 below.

**[Table 4]**

| | Bovine | | | | | | | | | |
|---|---|---|---|---|---|---|---|---|---|---|
| Monthly age | 74.02 | 52.12 | 54.08 | 52.27 | 65.3 | 24.25 | 57.26 | 83.08 | 33.08 | 62.05 |
| PYD | 198.80 12 | 324.70 35 | 263.59 64 | 164.51 67 | 318.77 23 | 188.27 68 | 361.93 12 | 227.52 85 | 279.25 32 | 358.36 8 |
| PEN | 0.1568 65 | 0.2385 82 | 0.1973 56 | 0.1445 44 | 0.4271 25 | 0.1571 96 | 0.2944 26 | 0.2489 95 | 0.2457 61 | 0.1802 57 |
| T-GLYC | 3.1621 02 | 3.8991 32 | 2.9629 2 | 1.8841 11 | 3.9474 06 | 3.1791 86 | 3.7058 87 | 3.1036 2 | 3.4969 58 | 4.5743 91 |
| PpP | 1267.3 36 | 1360.9 73 | 1335.6 37 | 1138.1 8 | 746.32 15 | 1197.7 21 | 1229.2 76 | 913.78 69 | 1136.2 79 | 1988.0 9 |
| PpG | 62.869 97 | 83.275 84 | 88.965 05 | 87.317 98 | 80.754 89 | 59.221 69 | 97.663 86 | 73.310 69 | 79.856 04 | 78.342 24 |

| | Bovine | | | | | | | | |
|---|---|---|---|---|---|---|---|---|---|
| Monthly age | 57.19 | 35.07 | 47.11 | 57.26 | 33.14 | 32.23 | 47.14 | 44.03 | 30.28 |
| PYD | 273.17 74 | 270.82 97 | 225.84 46 | 229.81 52 | 213.59 05 | 215.25 45 | 171.84 14 | 270.20 89 | 283.73 18 |
| PEN | 0.1854 39 | 0.1992 07 | 0.1825 43 | 0.1999 49 | 0.1391 1 | 0.1459 12 | 0.1423 28 | 0.1683 09 | 0.1724 13 |
| T-GLYC | 2.8264 7 | 2.8907 | 2.8269 87 | 3.0256 75 | 2.6672 72 | 2.7673 63 | 2.2694 92 | 2.4965 72 | 3.1007 1 |
| PpP | 1473.1 41 | 1359.5 42 | 1237.2 14 | 1149.3 69 | 1535.4 11 | 1475.2 36 | 1207.3 61 | 1605.4 36 | 1645.6 48 |
| PpG | 96.649 68 | 93.689 99 | 79.888 82 | 75.955 03 | 80.078 27 | 77.783 27 | 75.718 01 | 108.23 2 | 91.505 42 |

As shown in Fig.1, it has been made clear that as compared with pyridinoline content (ng/mg) of collagen contained in teeth of human non-diabetic patients and bovines, pyridinoline content (ng/mg) of collagen contained in teeth of human diabetic patients has increased somewhat more in accordance with monthly ages.

Additionally, as shown in Fig.2, it has been made clear that as compared with pentosidine content (ng/mg) of collagen contained in teeth of human non-diabetic patients and bovines, pentosidine content (ng/mg) of collagen contained in teeth of human diabetic patients has increased more in accordance with monthly ages.

Additionally, as shown in Fig.3, it has been made clear that as compared with total glycation product content (ng/mg: converted to pentosidine content) of collagen contained in teeth of human non-diabetic patients and bovines, total glycation product content (ng/mg: converted to pentosidine content) of collagen contained in teeth of human diabetic patients has increased more in accordance with monthly ages.

Additionally, as shown in Fig.4, it has been made clear that as compared with a PpP (pyridinoline content (ng/mg)/ pentosidine content (ng/mg)) being a value of a weight ratio calculated from a PYD being a quantitative value of pyridinoline content (ng/mg) of collagen contained in teeth of human non-diabetic patients and bovines, and a PEN being a quantitative value of pentosidine content (ng/mg) thereof, a PpP (pyridinoline content (ng/mg)/ pentosidine content (ng/mg)) being a value of a weight ratio calculated from a PYD being a quantitative value of pyridinoline content (ng/mg) of collagen contained in teeth of human diabetic patients and a PEN being a quantitative value of pentosidine content (ng/mg) thereof has decreased more in accordance with monthly ages.

Furthermore, as shown in Fig.5, it has been made clear that as compared with a PpG (pyridinoline content (ng/mg)/ total glycation product content (ng/mg: converted to pentosidine content)) being a value of a weight ratio calculated from a PYD being a quantitative value of pyridinoline content (ng/mg) of collagen contained in teeth of human non-diabetic patients and bovines, and a T-GLYC being a quantitative value of total glycation product content (ng/mg: converted to pentosidine content) thereof, a PpG (pyridinoline content (ng/mg)/ total glycation product content (ng/mg: converted to pentosidine content)) being a value of a weight ratio calculated from a PYD being a quantitative value of pyridinoline content (ng/mg) of collagen contained in teeth of human diabetic patients and a T-GLYC being a quantitative value of total glycation product content (ng/mg: converted to pentosidine content) thereof has decreased more in accordance with monthly ages.

Since it is clear, from the above, that these matters can be used as an index for evaluating a potential risk of developing a disease in a mammal and/or whether the mammal is potentially healthy, it has been shown that a method like a method according to the present invention, of using, as an index for evaluating a potential risk of developing a disease in a mammal and/or whether the mammal is potentially healthy, a quantitative value of pyridinoline content of collagen contained in teeth of a mammal is effective, that a method like a method according to the present invention, of using, as an index for evaluating a potential risk of developing a disease in a mammal and/or whether the mammal is potentially healthy, a quantitative value of pentosidine content of collagen contained in teeth of a mammal is effective, that a method like a method according to the present invention, of using, as an index for evaluating a potential risk of developing a disease in a mammal and/or whether the mammal is potentially healthy, a quantitative value of total glycation product content of collagen contained in teeth of a mammal is effective, that a method like a method according to the present invention, of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of pentosidine content thereof is effective, as well as that a method like a method according to the present invention, of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of total glycation product content thereof is effective.

## Claims

1. A method which is a method of using, as an index for evaluating a potential risk of developing a disease in a mammal and/or whether the mammal is potentially healthy, a quantitative value of pyridinoline content of collagen contained in teeth of a mammal,
and which includes
a process step of obtaining a PYD being a quantitative value by quantifying pyridinoline content (ng/mg) of collagen contained in teeth of a mammal to be evaluated for a potential risk of developing a disease and/or whether the mammal is potentially healthy,
a process step of obtaining an R-PYD being a reference quantitative value by quantifying pyridinoline content (ng/mg) of collagen contained in teeth of one or more mammals selected from a mammal that has not developed a disease, a mammal that has developed a disease, a healthy mammal, and a non-healthy mammal,
and a process step of comparing said PYD with said R-PYD.

2. A method which is a method of using, as an index for evaluating a potential risk of developing a disease in a mammal and/or whether the mammal is potentially healthy, a quantitative value of pentosidine content of collagen contained in teeth of a mammal,
and which includes
a process step of obtaining a PEN being a quantitative value by quantifying pentosidine content (ng/mg) of collagen contained in teeth of a mammal to be evaluated for a potential risk of developing a disease and/or whether the mammal is potentially healthy,
a process step of obtaining an R-PEN being a reference quantitative value by quantifying pentosidine content (ng/mg) of collagen contained in teeth of one or more mammals selected from a mammal that has not developed a disease, a mammal that has developed a disease, a healthy mammal, and a non-healthy mammal,
and a process step of comparing said PEN with said R-PEN.

3. A method which is a method of using, as an index for evaluating a potential risk of developing a disease in a mammal and/or whether the mammal is potentially healthy, a quantitative value of total glycation product content of collagen contained in teeth of a mammal,
and which includes
a process step of obtaining a T-GLYC being a quantitative value by quantifying total glycation product content (ng/mg: converted to pentosidine content) of collagen contained in teeth of a mammal to be evaluated for a potential risk of developing a disease and/or whether the mammal is potentially healthy,
a process step of obtaining an R-T-GLYC being a reference quantitative value by quantifying total glycation product content (ng/mg: converted to pentosidine content) of collagen contained in teeth of one or more mammals selected from a mammal that has not developed a disease, a mammal that has developed a disease, a healthy mammal, and a non-healthy mammal,
and a process step of comparing said T-GLYC with said R-T-GLYC.

4. A method which is a method of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of pentosidine content thereof,
and which includes
a process step of obtaining a PYD being a quantitative value by quantifying pyridinoline content (ng/mg) of collagen contained in teeth of a mammal to be evaluated for a potential risk of developing a disease and/or whether the mammal is potentially healthy,
a process step of obtaining a PEN being a quantitative value by quantifying pentosidine content (ng/mg) of collagen contained in teeth of said mammal to be evaluated,
a process step of calculating, from said PYD obtained and said PEN obtained, a PpP (pyridinoline content (ng/mg)/ pentosidine content (ng/mg)) being a value of a weight ratio,
a process step of obtaining an R-PYD being a reference quantitative value by quantifying pyridinoline content (ng/mg) of collagen contained in teeth of one or more mammals selected from a mammal that has not developed a disease, a mammal that has developed a disease, a healthy mammal, and a non-healthy mammal,
a process step of obtaining an R-PEN being a reference quantitative value by quantifying pentosidine content (ng/mg) of collagen contained in teeth of said one or more mammals,
a process step of calculating, from said R-PYD obtained and said R-PEN obtained, an R-PpP (pyridinoline content (ng/mg)/ pentosidine content (ng/mg)) being a reference value of a weight ratio,
and a process step of comparing said PpP with said R-PpP.

5. A method which is a method of using, as an index for evaluating a potential risk of developing a disease in a mammal and/or whether the mammal is potentially healthy, a value of a weight ratio calculated from a quantitative value of pyridinoline content of collagen contained in teeth of a mammal and a quantitative value of total glycation product content thereof,
and which includes
a process step of obtaining a PYD being a quantitative value by quantifying pyridinoline content (ng/mg) of collagen contained in teeth of a mammal to be evaluated for a potential risk of developing a disease and/or whether the mammal is potentially healthy,
a process step of obtaining a T-GLYC being a quantitative value by quantifying total glycation product content (ng/mg: converted to pentosidine content) of collagen contained in teeth of said mammal to be evaluated,
a process step of calculating, from said PYD obtained and said T-GLYC obtained, a PpG (pyridinoline content (ng/mg)/ total glycation product content (ng/mg: converted to pentosidine content)) being a value of a weight ratio,
a process step of obtaining an R-PYD being a reference quantitative value by quantifying pyridinoline content (ng/mg) of collagen contained in teeth of one or more mammals selected from a mammal that has not developed a disease, a mammal that has developed a disease, a healthy mammal, and a non-healthy mammal,
a process step of obtaining an R-T-GLYC being a reference quantitative value by quantifying total glycation product content (ng/mg: converted to pentosidine content) of collagen contained in teeth of said one or more mammals,
a process step of calculating, from said R-PYD obtained and said R-T-GLYC obtained, an R-PpG (pyridinoline content (ng/mg)/ total glycation product content (ng/mg: converted to pentosidine content)) being a reference value of a weight ratio,
and a process step of comparing said PpG with said R-PpG.

6. A method according to Claim 1, wherein a process step of carrying out quantification is a process step of carrying out quantification by utilizing a high-performance liquid chromatograph with a fluorescence detector (HPLC-Flu) using heptafluorobutyric acid and formic acid as an ion-pairing reagent.

7. A method according to any one claim of Claim 1 to Claim 6, wherein said mammals are one or more mammals selected from a group consisting of humans, bovines, horses, swine, wild boars, sheep, deer, dogs, and cats.
